# EUROPEAN PATENT APPLICATION

(11) **EP 4 559 519 A1**
(43) Date of publication of application: **28.05.2025**
(21) Application number: 23212155.8
(22) Date of filing: 25.11.2023
(51) Int. Cl.: A61P 1/00, A61P 11/00, C12N 9/24

(54) **MUTATED HUMAN ACIDIC CHITINASE FOR MEDICAL USE**

(71) Applicant: BIOINOVA, A.S., 14200 Praha 4 (CZ); Kogakuin University, Tokyo 163-8677 (JP)
(72) Inventor: Oyama, Fumitaka, Tokyo (JP); Bauer, Peter O., Praha 4 (CZ); Okawa, Kazuaki, Tokyo (JP)
(74) Representative: Harber IP s.r.o.

(57) **Abstract**

The present invention provides a mutated human acidic chitinase having amino acid sequence of the wild type human Chia and bearing the following mutations: R61M, M162T, I173S, M179L, N190T, V246A, D252N, N270T, I272L, and I300F. These mutations hyperactivate the acidic chitinase which is particularly suitable for medical uses.

## Description

### Field of Art

The present invention relates to human acidic chitinase, suitable for use in treatment of diseases.

### Background Art

Chitinase is an enzyme that catalyzes the hydrolysis of chitin, a polymer composed of N-acetyl-D-glucosamine, serving as a key structural component in the exoskeletons of crustaceans and insects, the microfilaria sheath of parasitic nematodes, and the cell walls of fungi. In both mice and humans, two chitinases with chitin-degrading capabilities are found: chitotriosidase (Chit1) and acidic chitinase (Chia), also known as acidic mammalian chitinase or AMCase. Chia, encoded by the Chia gene, earned its name due to its acidic isoelectric point.

Chia is highly expressed in the mouse stomach. Accordingly, a robust peak of activity is observed at pH 2.0, suggesting its function as a digestive enzyme that breaks down chitin in the stomach in omnivorous animals. In addition, recent genomic and evolutionary studies have shown that multiple Chia paralogs emerged based on insect intake, suggesting the possibility of mammalian and bird Chia being able to digest chitin contained in insects.

Chia has attracted attention due to its altered expression under diverse pathological conditions such as asthma, allergic lung inflammation, ocular allergy, and gastric cancer ( Zhu, Z. et al. (2004) Acidic mammalian chitinase in asthmatic Th2 inflammation and IL-13 pathway activation, Science. 304, 1678-82; Reese, T. A. et al. (2007) Chitin induces accumulation in tissue of innate immune cells associated with allergy, Nature. 447, 92-6; Bucolo, C. et al. (2008) Effect of chitinase inhibitors on endotoxin-induced uveitis (EIU) in rabbits, Pharmacol Res. 57, 247-52; Musumeci, M. et al. (2009) Acidic mammalian chitinase in dry eye conditions, Cornea. 28, 667-72; Bucolo, C. et al. (2011) Acidic mammalian chitinase and the eye: implications for ocular inflammatory diseases, Front Pharmacol. 2, 43; Cozzarini, E. et al. (2009) CHIT1 and AMCase expression in human gastric mucosa: correlation with inflammation and Helicobacter pylori infection, Eur J Gastroenterol Hepatol. 21, 1119-26; Nookaew, I. et al. (2013) Transcriptome signatures in Helicobacter pylori-infected mucosa identifies acidic mammalian chitinase loss as a corpus atrophy marker, BMC Med Genomics. 6, 41). Several genetic variants in Chia were associated with human bronchial asthma (Bierbaum, S. et al. (2005) Polymorphisms and haplotypes of acid mammalian chitinase are associated with bronchial asthma, Am J Respir Crit Care Med. 172, 1505-9; Seibold, M. A. et al. (2009) Differential enzymatic activity of common haplotypic versions of the human acidic mammalian chitinase protein, J Biol Chem. 284, 19650-8; Okawa, K. et al. (2016) Loss and gain of human acidic mammalian chitinase activity by nonsynonymous SNPs, Mol Biol Evol. 33, 3183-3193).

Environmental exposure to chitin from mites and molds, along with Chia's chitinase activity, has been associated with lung diseases (Van Dyken, S. J. et al. (2014) Chitin activates parallel immune modules that direct distinct inflammatory responses via innate lymphoid type 2 and gammadelta T cells, Immunity. 40, 414-24**;** Kim, L. K. et al. (2015) AMCase is a crucial regulator of type 2 immune responses to inhaled house dust mites, Proc Natl AcadSci U S A. 112, E2891-9). Chitin accumulated in the airways of Chia-deficient mice was degraded by transgenically expressed Chia, blocking the sustained stimulation of the inflammatory pathways associated with lung disease and ameliorating associated symptoms (Fitz, L. J. et al. (2012) Acidic mammalian chitinase is not a critical target for allergic airway disease, Am J Respir Cell Mol Biol. 46, 71-9; Van Dyken, S. J. et al. (2017) Spontaneous chitin accumulation in airways and age-related fibrotic lung disease, Cell. 169, 497-509 e13). Thus, chitinase activity may strongly affect human pulmonary asthma and fibrosis. Therefore, active Chia can improve symptoms of these diseases (Van Dyken, S. J. et al. (2017) Spontaneous chitin accumulation in airways and age-related fibrotic lung disease, Cell. 169, 497-509 e13; Barad, B. A. et al. (2020) Differences in the chitinolytic activity of mammalian chitinases on soluble and insoluble substrates, Protein Sci. 29, 966-977).

The chitinase activity of human Chia is lower than that of mouse Chia. Human wild-type (WT) Chia has an optimum pH of 5.0 and an activity of 1/50 that of the mouse (Goedken, E. R. et al. (2011) Functional comparison of recombinant acidic mammalian chitinase with enzyme from murine bronchoalveolar lavage, Protein Expr Purif. 75, 55-62). However, it has been reported that substituting R61 with methionine in human Chia shifted the optimum to pH 2.0 and resulted in a level of activation similar to that in mice (Okawa, K. et al. (2016) Loss and gain of human acidic mammalian chitinase activity by nonsynonymous SNPs, Mol Biol Evol. 33, 3183-3193). WO2018191379 discloses modified Chia with increased chitinase activity.

Further improvements in the activity of the human Chia would be desirable to achieve an acceptable therapeutic activity.

### Disclosure of the Invention

The present invention relates to human acidic chitinase (Chia) bearing the following mutations in comparison to wild-type Chia: R61M, M162T, I173S, M179L, N190T, V246A, D252N, N270T, I272L, and I300F.

Human acidic chitinase (wild-type Chia) (EC 3.2.1.14) is an enzyme containing 476 amino acids. It is a member of the glycosyl hydrolase 18 family. The human Chia cDNA sequence was first reported by Boot et al. (Boot, R. G., Blommaart, E. F., Swart, E., Ghauharali-van der Vlugt, K., Bijl, N., Moe, C., Place, A. & Aerts, J. M. (2001) Identification of a novel acidic mammalian chitinase distinct from chitotriosidase, J Biol Chem. 276, 6770-8) as GenBankTM accession number AF290004 (protein_ID AAG60019.1). It is now registered NP_970615.2 as Gene ID: 27159 in the NCBI database.

The human wild-type Chia amino acid sequence is as follows: 







 (SEQ ID NO. 1),
 wherein the underlined N-terminal 21-amino acid sequence represents the signal sequence. The mature Chia sequence begins in position 22.

It has been reported that there are 8 nonsynonymous single nucleotide polymorphisms in wild-type human Chia: A290G (N45D), G296A (D47N), G339T (R61M), G461A (G102R), A531G (K125R), G1172A (V339I), T1218C (F354S), and G1452T (G432V) (Seibold, M. A. et al (2009) Differential enzymatic activity of common haplotypic versions of the human acidic mammalian chitinase protein, J Biol Chem. 284, 19650-8). These polymorphisms are hereby included in the definition of the wild-type human Chia.

In one aspect, object of the invention is human acidic chitinase (human Chia) bearing the following mutations: R61M, M162T, I173S, M179L, N190T, V246A, D252N, N270T, I272L, and I300F.

Preferably, object of the invention is human acidic chitinase (human Chia) having amino acid sequence of the wild type human Chia and bearing the following mutations: R61M, M162T, I173S, M179L, N190T, V246A, D252N, N270T, I272L, and I300F, wherein the sequence outside the mutations listed herein above has at least 95% identity, preferably at least 98% identity, to the wild type human Chia (SEQ ID NO. 1).

In an aspect of the invention, the human Chia, according to the invention, contains an amino acid sequence having at least 95% identity, preferably at least 98% identity, to the sequence SEQ ID NO. 3: 
YQLTCYFTNWAQYRPGLGRFMPDNIDPCLCTHLIYAFAGMQNNEITTIEWNDVTLYQAFNGL
KNKNSQLKTLLAIGGWNFGTAPFTAMVSTPENRQTFITSVIKFLRQYEFDGLDFDWEYPGSRG
SPPQDKHLFTVLVQETREAFEQEAKQSNKPRLLVTAAVAAGISTIQSGYEIPQLSQYLDYIHVM
TYDLHGSWEGYTGENSPLYKYPTDTGSNAYLNVDYAMNYWKNNGAPAEKLIVGFPTYGHT
FLLSNPSNTGIGAPTSGAGPAGPYAKESGFWAYYEICTFLKNGATQGWDAPQEVPYAYQGNV
WVGYDNIKSFDIKAQWLKHNKFGGAMVWAIDLDDFTGTFCNQGKFPLISTLKKALGLQSAS
CTAPAQPIEPITAAPSGSGNGSGSSSSGGSSGGSGFCAVRANGLYPVANNRNAFWHCVNGVT
YQQNCQAGLVFDTSCDCCNWA (SEQ ID NO. 3),
wherein the underlined amino acids are conserved, and
wherein the SEQ ID NO. 3 must be interpreted as including the natural polymorphisms N45D, D47N, G102R, K125R, V339I, F354S, and G432V, wherein the numbering of the positions corresponds to wild-type human Chia sequence including the signal sequence MTKLILLTGLVLILNLQLGSA (SEQ ID NO. 4) on N-terminus.

In some embodiments, the human acidic chitinase of the invention has an amino acid sequence having at least 95% identity, preferably at least 98% identity, to the sequence: 
MTKLILLTGLVLILNLQLGSAYQLTCYFTNWAQYRPGLGRFMPDNIDPCLCTHLIYAFAGMQ
NNEITTIEWNDVTLYQAFNGLKNKNSQLKTLLAIGGWNFGTAPFTAMVSTPENRQTFITSVIK
FLRQYEFDGLDFDWEYPGSRGSPPQDKHLFTVLVQETREAFEQEAKQSNKPRLLVTAAVAAG
ISTIQSGYEIPQLSQYLDYIHVMTYDLHGSWEGYTGENSPLYKYPTDTGSNAYLNVDYAMNY
WKNNGAPAEKLIVGFPTYGHTFLLSNPSNTGIGAPTSGAGPAGPYAKESGFWAYYEICTFLKN
GATQGWDAPQEVPYAYQGNVWVGYDNIKSFDIKAQWLKHNKFGGAMVWAIDLDDFTGTF
CNQGKFPLISTLKKALGLQSASCTAPAQPIEPITAAPSGSGNGSGSSSSGGSSGGSGFCAVRAN
GLYPVANNRNAFWHCVNGVTYQQNCQAGLVFDTSCDCCNWA (SEQ ID NO. 2)
wherein the underlined amino acids in positions 61, 162, 173, 179, 190, 246, 252, 270, 272 and 300 are conserved, and
wherein the SEQ ID NO. 2 must be interpreted as including the natural polymorphisms N45D, D47N, G102R, K125R, V339I, F354S, and G432V.

The sequence identity is defined as the percentage of matches of the same amino acid residues between two aligned sequences. The sequence identity lower than 100% may be due to single-nucleotide mutations, wherein an amino acid is replaced by another naturally-occurring amino acid, preferably by a similar amino acid resulting in a conservative mutation. Conservative mutation is an amino acid replacement changing a given amino acid to a different amino acid with similar charge, hydrophobicity and/or size.

For example, conservative mutation is a substitution of one amino acid by another amino acid within one of the following classes:
- aliphatic: G, A, V, L and I;
- hydroxyl and/or sulfur containing: S, T and M;
- acidic/amides: D and N.

Preferably, the human acidic chitinase of the invention has an amino acid sequence having at least 98%, more preferably at least 99% identity to the sequence SEQ ID NO. 3 or SEQ ID NO. 2, wherein the amino acids in positions 61, 162, 173, 179, 190, 246, 252, 270, 272 and 300 are conserved (the numbering of the positions corresponds to wild-type human Chia including the signal sequence SEQ ID NO. 4). SEQ ID NO. 2 and SEQ ID NO. 3 must be interpreted as including the natural polymorphisms N45D, D47N, G102R, K125R, V339I, F354S, and G432V.

Most preferably, the human acidic chitinase of the invention has the amino acid sequence SEQ ID NO. 2. SEQ ID NO. 2 must be interpreted as including the natural polymorphisms N45D, D47N, G102R, K125R, V339I, F354S, and G432V.

All mutated human acidic chitinases of the invention have chitinase activity.

Within the framework of the present invention, it has been found that the amino acid substitutions R61M, M162T, I173S, M179L, N190T, V246A, D252N, N270T, I272L, and I300F, compared to wild-type human Chia, significantly increase the chitinase activity of the human Chia. The hyperactivated Chia of the invention exhibits excellent enzymatic properties, as well as high thermostability and high pH stability. A significant increase in chitinase activity was observed at pH 2.0, 5.0 and 7.0. The enzyme of the invention has a strong chitinolytic activity not only for low-molecular-weight substrates but also for high-molecular-weight colloidal chitin. At the same time, the 10 mutations represent a minimal genetic manipulation for human Chia activation.

Furthermore, the present invention provides the mutated human Chia of the invention, preferably the Chia having the sequence SEQ ID NO. 2 or containing SEQ ID NO. 3, for the treatment of a disease selected from: asthma, allergic lung inflammation, ocular allergy, gastric cancer, bronchial asthma, human pulmonary asthma and lung fibrosis.

Yet furthermore, the mutated human Chia of the invention, preferably the Chia having the sequence SEQ ID NO. 2 or containing SEQ ID NO. 3, is suitable for use as an antifungal agent *in vitro* and *in vivo.*

Yet furthermore, the mutated human Chia of the invention, preferably the Chia having the sequence SEQ ID NO. 2 or containing SEQ ID NO. 3, is suitable for use in digestive enzyme preparations for gastrointestinal applications in humans, to treat digestive disorders.

The mutated human Chia of the invention may be administered in the form of a pharmaceutical composition, comprising the mutated human Chia of the invention and at least one pharmaceutically acceptable auxiliary substance. Pharmaceutically acceptable auxiliary substances may be selected from solvents, fillers, binders, preservatives, glidants and the like. Suitable pharmaceutically acceptable auxiliary substances are known in the art of formulation. The solvents are preferably aqueous solvents, such as saline solution and buffers.

Formulation and administration methods may vary depending on the intended medical application:
- Airway Conditions (e.g., Asthma, Allergic Lung Inflammation): Consider droplets, sprays and inhalers with robust packaging to prevent premature degradation.
- Ophthalmology: Utilize eye drops with specialized packaging for sterility and controlled dispensing.
- Gastric Conditions (e.g., Gastric Cancer): Formulate tablets or capsules for oral administration, potentially with enteric coatings for protection.
- Antifungal Agent: Develop creams, ointments, or solutions suitable for topical or systemic use.
- Digestive Enzyme Preparation for GIT: Formulate tablets, capsules, or liquid solutions for oral intake.

### Brief description of drawings

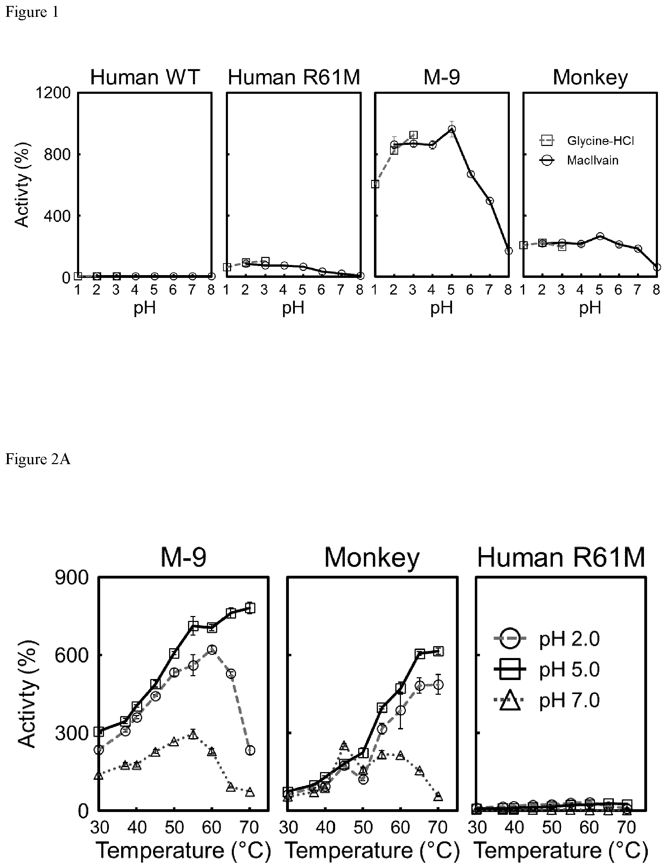

**Fig. 1****: Enhanced Chitinase Activity at Various pH Conditions.** Figure 1 illustrates the hyperactivation of human Chia by substituting 9 amino acids (+R61M) (M-9) compared to human R61M Chia at various pH conditions (human M-9 Chia is the mutated Chia of the invention, having the amino acid sequence SEQ ID NO. 2). Human R61M Chia at pH 5.0 is the reference (100%) and is presented as relative activity. The values in the graph represent the average values from three measurements, with error bars indicating the mean ± standard deviation from a single experiment conducted in triplicate.

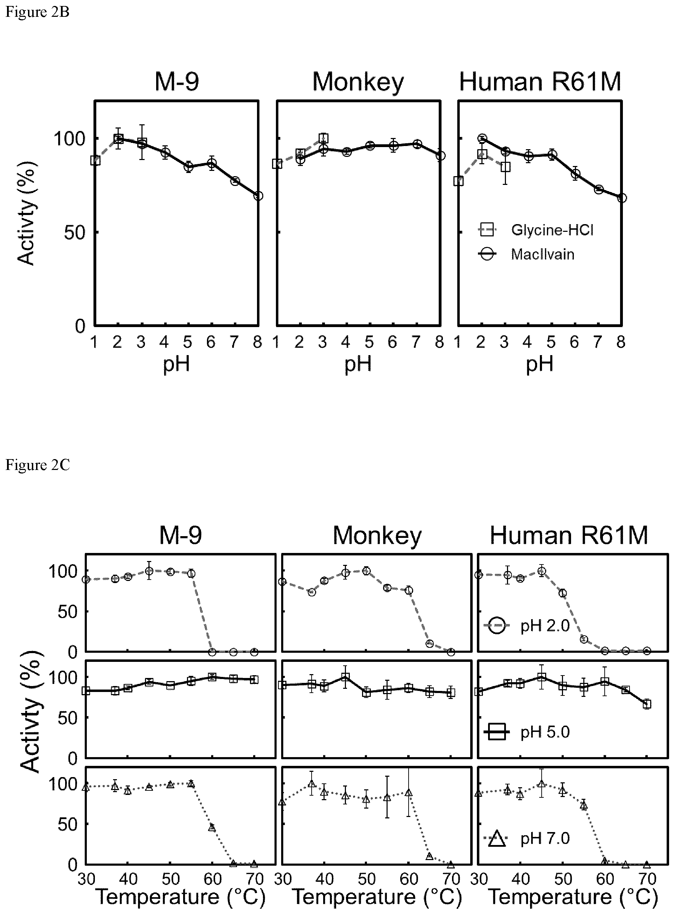

**Fig. 2****: Enzymatic Properties of M-9 Under Varied Conditions.** Figure 2 presents the enzymatic properties of M-9 (Chia having the amino acid sequence SEQ ID NO. 2) under various conditions. In Panel A (Figure 2A), the temperature dependence of M-9 at different pH levels is superior to that of the human Chia R61M. Reaction conditions are classified using geometric shapes (pH 2.0, 2,0 and 7.0). In Panel B (Figure 2B), pH stability at 37°C for each Chia protein is shown, with the maximum activity of each Chia set at 100% and the data representing relative activity. In Panel C (Figure 2C), the thermal stability of the Chia proteins is analyzed by pre-incubating samples at 30-70°C at each pH for 30 minutes, followed by measuring the residual activity at pH 5.0 and 37°C for 5 minutes. M-9 remains stable at all temperatures at pH 5.0. In contrast, at pH 7.0, a drop and absence of activity are observed at 60 and ≥65°C, respectively, similar to human R61M Chia. The maximum activity of each Chia is set as 100% and presented as relative activity.

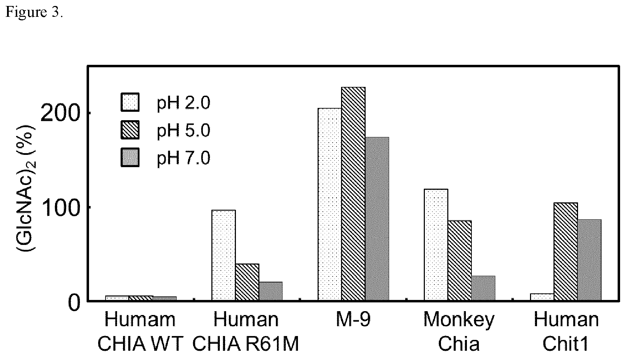

**Fig. 3****: Superior High-Molecular-Weight Colloidal Chitin (p-chitin) Degradation by M-9.** Figure 3 demonstrates the superior ability of M-9 to degrade high-molecular-weight colloidal chitin (p-chitin) compared to human WT and R61M Chia and human Chit1 enzymes. M-9 exhibits significantly higher chitinase activity against p-chitin across all pH conditions (analyzed by FACE method). The chart provides quantification data for (GlcNAc)₂, presented relative to the maximum amount of R61M Chia degradation products (pH 2.0), set at 100%.

### Examples of carrying out the invention

Materials and methods:

### Construction of human and monkey Chia expression vector for E. coli-expression

E. coli-expression vectors for human and monkey Chia were constructed [Uehara, M. et al. (2021) Robust chitinolytic activity of crab-eating monkey (Macaca fascicularis) acidic chitinase under a broad pH and temperature range, Sci Rep. 11, 15470, Okawa, K. et al. (2016) Loss and gain of human acidic mammalian chitinase activity by nonsynonymous SNPs, Mol Biol Evol. 33, 3183-3193] by PCR using KOD Plus DNA polymerase and oligonucleotide primers anchored with the restriction sites for BamHI and XhoI as described previously [Tabata, E. et al. (2022) Noninsect-based diet leads to structural and functional changes of acidic chitinase in Carnivora, Mol Biol Evol. 39, msab331]. The amplified cDNA was digested with BamHI and XhoI and then cloned into the pET22b/pre-Protein A-dog Chia-V5-His vector at the same site. The resulting plasmid DNA, pET22b/YKL-40/V5-His, was sequenced to confirm its complete sequence.

### Construction of Chimeric proteins and preparation of human Chia mutant proteins

Monkey/human chimeric proteins were constructed by fusing two units at the junctions of exons 3-5, exons 6-7, exons 8-10, and exon 11, as previously described [Tabata, E. et al. (2022) Noninsect-based diet leads to structural and functional changes of acidic chitinase in Carnivora, Mol Biol Evol. 39, msab331, Okawa, K. et al. (2016) Loss and gain of human acidic mammalian chitinase activity by nonsynonymous SNPs, Mol Biol Evol. 33, 3183-3193], since both molecules share similar exon structures at the nucleotide level. Human Chia mutant proteins were prepared by PCR using previously described primers [Tabata, E. et al. (2022) Noninsect-based diet leads to structural and functional changes of acidic chitinase in Carnivora, Mol Biol Evol. 39, msab331].

### Preparation of recombinant human and monkey Chia proteins expressed in E. coli

Preparation of Protein A-human Chia-V5-His and Protein A-monkey Chia-V5-His from the *E*. *coli* was performed and purified by IgG Sepharose as described previously [Tabata, E. et al. (2022) Noninsect-based diet leads to structural and functional changes of acidic chitinase in Carnivora, Mol Biol Evol. 39, msab331].

### SDS-Polyacrylamide Gel Electrophoresis and Western Blot

The protein fractions were analyzed using standard SDS-polyacrylamide gel electrophoresis (PAGE), followed by Western blot using an anti-V5-HRP monoclonal antibody.

### Chitinase enzymatic assays

Chitinase enzyme activity was determined with the fluorogenic substrate 4-MU β-D- *N, N'-*diacetylchitobioside hydrate as a substrate in McIlvaine's buffer (0.1 M citric acid and 0.2 M Na₂HPO₄; pH 2.0, 5.0, 7.0) at 37°C for 30 min as described previously [Tabata, E. et al. (2022) Noninsect-based diet leads to structural and functional changes of acidic chitinase in Carnivora, Mol Biol Evol. 39, msab331]. The fluorescence of released 4-MU was measured using a Fluorometer with excitation at 365 nm and emission at 415-445 nm. The results are shown in Fig. 1 and 2.

### Analysis of Chitooligosaccharides by FACE

Colloidal chitin (1 mg/reaction) was incubated with recombinant Chia proteins at pH 2.0, 5.0 or 7.0 in McIlvaine's buffer (0.1 M citric acid and 0.2 M Na₂HPO₄) at 50°C for 1 h. The degradation products were labeled and separated by fluorophore-assisted carbohydrate electrophoresis (FACE). The results are shown in Fig. 3.

### Discussion:

Crab-eating monkeys (*Macaca fascicularis*) are a nonhuman primate animal model for biomedical research. As manifested by the name, they feed on crabs and other chitin-containing organisms, such as crustaceans and insects. Crab-eating monkey (monkey) Chia is highly homologous to human Chia, which is most active at pH 5.0 in the lungs, maintains its activity between pH 1.0 and 7.0, and at elevated temperatures (50°C-70°C). It is 16 times more active than mouse Chia at pH 5.0.

Through the strategy of chimeric proteins between human and monkey Chia proteins, some chimeric Chia exceeded the human and monkey enzymes' activity while maintaining their levels under neutral pH conditions. Introduction of nine amino acids present in monkey Chia into human R61M Chia to produce a mutated human Chia having the sequence SEQ ID NO. 2 (the protein is herein referred to as M-9) showed hyperactivation of the human enzyme, which has the potential for treating lung diseases. The enzymatic properties of the mutant M-9 were investigated and compared with the monkey and human R61M Chia.

The hyperactivated M-9 exhibited excellent enzymatic properties and thermostability as well as pH stability. M-9 had the highest activity at pH 5.0, followed by pH 2.0, and maintained its activity also at pH 7.0 to 8.0 (Fig. 1).

The activity of M-9 increased with increasing temperature, reaching the maximum at 70°C and 55°C at pH 5.0 (Fig. 2A). At pH 2.0, the maximum activity of M-9 was at 55°C. M-9 exhibited chitinase activity that exceeded parental chitinase activities even at pH 2.0, 5.0, and 7.0 under high-temperature conditions (Fig. 2A). M-9 is stable under acidic conditions (Fig. 2B). M-9 remained stable at 30-70°C at pH 5.0 (Fig. 2C).

Finally, we investigated the degradation of high molecular weight colloidal chitin (p-chitin) by M-9 and compared the enzymatic activity with other chitinases. M-9 showed higher chitinase activity for p-chitin than human Chia WT, R61M Chia, Chit1 and the monkey Chia at each pH 2.0, 5.0, and 7.0 (Fig. 3A and B). These results confirmed the high chitinase activity of M-9 against chitin substrate under physiological conditions exceeding those of other tested enzymes (Fig. 3).

## Claims

1. Mutated human acidic chitinase containing an amino acid sequence having at least 95% identity, preferably at least 98% identity, to the wild-type human Chia and additionally bearing the following mutations in comparison to wild-type human Chia: R61M, M162T, I173S, M179L, N190T, V246A, D252N, N270T, I272L, and I300F.

2. Mutated human acidic chitinase according to claim 1, containing an amino acid sequence having at least 95% identity, preferably at least 98% identity, to the sequence SEQ ID NO. 3: 
YQLTCYFTNWAQYRPGLGRFMPDNIDPCLCTHLIYAFAGMQNNEITTIEWNDVTLYQAFNGL
KNKNSQLKTLLAIGGWNFGTAPFTAMVSTPENRQTFITSVIKFLRQYEFDGLDFDWEYPGSRG
SPPQDKHLFTVLVQETREAFEQEAKQSNKPRLLVTAAVAAGISTIQSGYEIPQLSQYLDYIHVM
TYDLHGSWEGYTGENSPLYKYPTDTGSNAYLNVDYAMNYWKNNGAPAEKLIVGFPTYGHT
FLLSNPSNTGIGAPTSGAGPAGPYAKESGFWAYYEICTFLKNGATQGWDAPQEVPYAYQGNV
WVGYDNIKSFDIKAQWLKHNKFGGAMVWAIDLDDFTGTFCNQGKFPLISTLKKALGLQSAS
CTAPAQPIEPITAAPSGSGNGSGSSSSGGSSGGSGFCAVRANGLYPVANNRNAFWHCVNGVT
YQQNCQAGLVFDTSCDCCNWA (SEQ ID NO. 3),
wherein the underlined amino acids are conserved, and
wherein the SEQ ID NO. 3 is interpreted as including the natural polymorphisms N45D, D47N, G102R, K125R, V339I, F354S, and G432V, wherein the numbering of the positions corresponds to wild-type human Chia sequence including the signal sequence MTKLILLTGLVLILNLQLGSA (SEQ ID NO. 4) on N-terminus.

3. Mutated human acidic chitinase according to claim 1, containing the amino acid sequence SEQ ID NO. 3: 
YQLTCYFTNWAQYRPGLGRFMPDNIDPCLCTHLIYAFAGMQNNEITTIEWNDVTLYQAFNGL
KNKNSQLKTLLAIGGWNFGTAPFTAMVSTPENRQTFITSVIKFLRQYEFDGLDFDWEYPGSRG
SPPQDKHLFTVLVQETREAFEQEAKQSNKPRLLVTAAVAAGISTIQSGYEIPQLSQYLDYIHVM
TYDLHGSWEGYTGENSPLYKYPTDTGSNAYLNVDYAMNYWKNNGAPAEKLIVGFPTYGHT
FLLSNPSNTGIGAPTSGAGPAGPYAKESGFWAYYEICTFLKNGATQGWDAPQEVPYAYQGNV
WVGYDNIKSFDIKAQWLKHNKFGGAMVWAIDLDDFTGTFCNQGKFPLISTLKKALGLQSAS
CTAPAQPIEPITAAPSGSGNGSGSSSSGGSSGGSGFCAVRANGLYPVANNRNAFWHCVNGVT
YQQNCQAGLVFDTSCDCCNWA (SEQ ID NO. 3),
wherein the underlined amino acids are conserved, and
wherein the SEQ ID NO. 3 is interpreted as including the natural polymorphisms N45D, D47N, G102R, K125R, V339I, F354S, and G432V, wherein the numbering of the positions corresponds to wild-type human Chia sequence including the signal sequence MTKLILLTGLVLILNLQLGSA (SEQ ID NO. 4) on N-terminus.

4. Mutated human acidic chitinase according to claim 1, having an amino acid sequence having at least 95% identity, preferably at least 98% identity, to the sequence: 
MTKLILLTGLVLILNLQLGSAYQLTCYFTNWAQYRPGLGRFMPDNIDPCLCTHLIYAFAGMQ
NNEITTIEWNDVTLYQAFNGLKNKNSQLKTLLAIGGWNFGTAPFTAMVSTPENRQTFITSVIK
FLRQYEFDGLDFDWEYPGSRGSPPQDKHLFTVLVQETREAFEQEAKQSNKPRLLVTAAVAAG
ISTIQSGYEIPQLSQYLDYIHVMTYDLHGSWEGYTGENSPLYKYPTDTGSNAYLNVDYAMNY
WKNNGAPAEKLIVGFPTYGHTFLLSNPSNTGIGAPTSGAGPAGPYAKESGFWAYYEICTFLKN
GATQGWDAPQEVPYAYQGNVWVGYDNIKSFDIKAQWLKHNKFGGAMVWAIDLDDFTGTF
CNQGKFPLISTLKKALGLQSASCTAPAQPIEPITAAPSGSGNGSGSSSSGGSSGGSGFCAVRAN
GLYPVANNRNAFWHCVNGVTYQQNCQAGLVFDTSCDCCNWA (SEQ ID NO. 2)
wherein the underlined amino acids in positions 61, 162, 173, 179, 190, 246, 252, 270, 272 and 300 are conserved, and
wherein the SEQ ID NO. 2 is interpreted as including the natural polymorphisms N45D, D47N, G102R, K125R, V339I, F354S, and G432V.

5. Mutated human acidic chitinase according to claim 1, having the amino acid sequence SEQ ID NO. 2: 
MTKLILLTGLVLILNLQLGSAYQLTCYFTNWAQYRPGLGRFMPDNIDPCLCTHLIYAFAGMQ
NNEITTIEWNDVTLYQAFNGLKNKNSQLKTLLAIGGWNFGTAPFTAMVSTPENRQTFITSVIK
FLRQYEFDGLDFDWEYPGSRGSPPQDKHLFTVLVQETREAFEQEAKQSNKPRLLVTAAVAAG
ISTIQSGYEIPQLSQYLDYIHVMTYDLHGSWEGYTGENSPLYKYPTDTGSNAYLNVDYAMNY
WKNNGAPAEKLIVGFPTYGHTFLLSNPSNTGIGAPTSGAGPAGPYAKESGFWAYYEICTFLKN
GATQGWDAPQEVPYAYQGNVWVGYDNIKSFDIKAQWLKHNKFGGAMVWAIDLDDFTGTF
CNQGKFPLISTLKKALGLQSASCTAPAQPIEPITAAPSGSGNGSGSSSSGGSSGGSGFCAVRAN
GLYPVANNRNAFWHCVNGVTYQQNCQAGLVFDTSCDCCNWA (SEQ ID NO. 2)
wherein the underlined amino acids in positions 61, 162, 173, 179, 190, 246, 252, 270, 272 and 300 are conserved, and
wherein the SEQ ID NO. 2 is interpreted as including the natural polymorphisms N45D, D47N, G102R, K125R, V339I, F354S, and G432V.

6. Mutated human acidic chitinase according to any one of claims 1 to 5 for use in the treatment of a disease selected from asthma, allergic lung inflammation, ocular allergy, gastric cancer, bronchial asthma, human pulmonary asthma and lung fibrosis.

7. Mutated human acidic chitinase according to any one of claims 1 to 5 for use as an antifungal agent in the treatment of fungal diseases.

8. Use of mutated human acidic chitinase according to any one of claims 1 to 5 as an antifungal agent in vitro.

9. Mutated human acidic chitinase according to any one of claims 1 to 5 for use in digestive enzyme preparations for gastrointestinal applications in humans, to treat digestive disorders.

10. A pharmaceutical composition, comprising the mutated human acidic chitinase according to any one of claims 1 to 5 and at least one pharmaceutically acceptable auxiliary substance, preferably the pharmaceutically acceptable auxiliary substance is selected from solvents, fillers, binders, preservatives, glidants.

11. The pharmaceutical composition according to claim 10, wherein the pharmaceutically acceptable auxiliary substance is aqueous saline solution or aqueous buffer.
